# EUROPÄISCHE PATENTSCHRIFT

(11) **EP 3 413 712 B1**
(45) Veröffentlichungstag und Bekanntmachung des Hinweises auf die Patenterteilung: **07.07.2021**
(21) Anmeldenummer: 17708431.6
(22) Anmeldetag: 08.02.2017
(51) Int. Cl.: A01N 37/36, A01N 25/22, A61L 2/22

(54) **DESINFEKTIONSMITTEL**
DISINFECTION AGENT
AGENT DE DÉSINFECTION

(30) Priorität: 12.02.2016 DE 102016102463
(43) Veröffentlichungstag der Anmeldung: 19.12.2018
(73) Patentinhaber: Mavena International AG, 6331 Hünenberg (CH)
(72) Erfinder: REICHWAGEN, Sven, 59227 Ahlen (DE)
(74) Vertreter: Schneiders & Behrendt Bochum
(86) Internationale Anmeldenummer: PCT/EP2017/052684
(87) Internationale Veröffentlichungsnummer: WO 2017/137408

(56) Entgegenhaltungen:
- EP-A1- 0 487 994
- WO-A1-01/52827
- WO-A1-2006/076334
- WO-A1-2008/088873
- WO-A1-2011/005270
- WO-A2-2006/125657
- WO-A2-2008/016837
- DE-C- 968 992
- US-A1- 2003 152 528
- US-A1- 2014 004 208
- VIMONT ALLISON ET AL: "Study of the Virucidal Potential of Organic Peroxyacids Against Norovirus on Food-Contact Surfaces", FOOD AND ENVIRONMENTAL VIROLOGY, SPRINGER US, BOSTON, Bd. 7, Nr. 1, 22. November 2014 (2014-11-22), Seiten 49-57, XP035442936, ISSN: 1867-0334, DOI: 10.1007/S12560-014-9174-0 [gefunden am 2014-11-22]
- DATABASE WPI Week 200639 Thomson Scientific, London, GB; AN 2006-373683 XP002768551, -& CN 1 711 845 A (YIN S) 28. Dezember 2005 (2005-12-28)
- .: "Polysorbate 20", The United States Pharmacopeial Convention, 1 September 2014 (2014-09-01), pages 1-1, XP55609584, U.S.A. Retrieved from the Internet: URL:https://www.uspnf.com/sites/default/fi les/usp_pdf/EN/USPNF/m66800.pdf [retrieved on 2019-07-30]
- .: "Polysorbate 20 (01/2008:426)", European Pharmacopeia 6.0, 1 January 2008 (2008-01-01), pages 2709-2710, XP55609588, Strasbourg, France Retrieved from the Internet: URL:http://www.uspbpep.com/ep60/polysorbat e%2020%200426e.pdf [retrieved on 2019-07-30]

## Beschreibung

Die Erfindung betrifft ein Mittel zum Desinfizieren von Körperoberflächen und Zerstören von bakteriellen Stoffwechselprodukten auf der Haut, das wenigstens eine Hydroxypercarbonsäure zusammen mit einem Stabilisierungsmittel in wässriger Lösung enthält.

Die Verwendung von Percarbonsäuren zu Desinfektionszwecken ist grundsätzlich bekannt, beispielsweise aus der EP 0 677 990 B1. Insbesondere ist dort die Verwendung von Peressigsäure beschrieben. Das Desinfektionsmittel eignet sich unter anderem zur Behandlung von medizinischen Ausrüstungsgegenständen, beispielsweise Endoskopen.

Die Herstellung von Percarbonsäurelösungen erfolgt durch Umsetzung der Carbonsäure mit Wasserstoffperoxid in wässrigem Medium in Anwesenheit eines Katalysators, üblicherweise Schwefelsäure. Bei der Reaktion bildet sich ein Gleichgewicht zwischen der Percarbonsäure als Reaktionsprodukt einerseits und den Edukten, d.h. der Carbonsäure und Wasserstoffperoxid andererseits, aus. Häufig wird nach erfolgter Reaktion das Reaktionsgemisch mit einem Lösemittel weiter verdünnt, um die gewünschte niedrigere Percarbonsäurekonzentration einzustellen. So können etwa für Toilettenreinigungszwecke Lösungen eingesetzt werden, die 0,5 bis 1 Gew.-% Peressigsäure enthalten. In der Regel wird zunächst die Percarbonsäure in höher konzentrierter Lösung hergestellt, da andernfalls die Gleichgewichtseinstellung einen zu langen Zeitraum in Anspruch nehmen würde.

Auch bei Verdünnung einer konzentrierten Gleichgewichtslösung der Percarbonsäure mit Wasser verändert sich der Gleichgewichtspunkt des Systems zugunsten der Regenerierung der ursprünglichen Reaktanten, wobei auch diese Gleichgewichtseinstellung einen langen Zeitraum von mehreren Wochen oder Monaten in Anspruch nehmen kann. Entsprechend handelt es sich bei der verdünnten Percarbonsäurelösung um eine Lösung veränderlicher Zusammensetzung, die aufgrund der Zersetzung von Wasserstoffperoxid mehr und mehr ihre Wirkung verliert.

Aus der WO 2006/125 657 A2, auf deren Inhalt ausdrücklich Bezug genommen wird, ist ein wässriges Desinfektions-und Entkeimungsmittel bekannt, bei dem Percarbonsäuren durch den Zusatz von Sorbitanestern stabilisiert sind. Diese Methode hat sich als grundsätzlich erfolgreich erwiesen. Die Verwendung von Sorbitanestern schränkt aber den Einsatzbereich derartig stabilisierter Percarbonsäuren, vor allem im medizinischen und Pflegebereich, ein.

Aus der WO 2006/076334 A1 geht eine mikrobiozide Zusammensetzung auf Basis von Wasserstoffperoxid, Peroxyessigsäure und Polyvinylpyrrolidon hervor. A. Vimont et al., Food and Environmental Virology (2015), 49-57, befasst sich mit dem viruziden Potential organischer Peroxysäuren. Die DE 968 992 C beschreibt mit Polyvinylpyrrolidon stabilisierte Wasserstoffperoxidlösungen.

Ausgehend von diesem Stand der Technik stellt sich die Aufgabe, ein alternatives Stabilisierungsmitteln für Hydroxypercarbonsäuren bereitzustellen, das die Anwendung damit stabilisierter Desinfektionsmittel insbesondere auch im medizinischen und Pflegebereich ermöglicht. Ein solches Stabilisierungsmittel sollte gut hautverträglich sein und Hydroxypercarbonsäuren für eine nachhaltige Wirkung hinreichend lang auf der Haut stabilisieren.

Diese Aufgabe wird mit einem Mittel der eingangs genannten Art gelöst, das als Stabilisierungsmittel Polyvinylpyrrolidon (PVP) enthält. Der Erfindung liegt die Erkenntnis zugrunde, dass die Zugabe einer signifikanten Menge Polyvinylpyrrolidon die Stabilität der Hydroxypercarbonsäuren erheblich erhöht. Es wurde gefunden, dass sich die Percarbonsäurekonzentration über einen langen Zeitraum praktisch nicht verändert, so dass sich eine Verwendbarkeit des Mittels über Monate oder Jahre ergibt. Der Effekt wird darauf zurückgeführt, dass Polyvinylpyrrolidon die Persäurefunktion komplexiert und dadurch die Abspaltung von Sauerstoff verhindert. Gleichzeitig wird die Aggressivität kurzkettiger Percarbonsäuren vermindert, so dass sie für die Anwendung auf der Haut infrage kommen.

Zudem ist Polyvinylpyrrolidon ein in der Pharmazie und Lebensmittelindustrie eingeführtes und vielfach verwandtes Produkt, das gut vertragen wird und hautfreundlich ist. In Lebensmitteln wird es als Binde-und Fertigungsmittel eingesetzt, in Tabletten als Bindemittel und in der Wundbehandlung als Trägerstoff für Jod.

Polyvinylpyrrolidon, auch unter der Bezeichnung Povidon bekannt und vielfach mit PVP abgekürzt, ist ein sehr gut wasserlösliches Polymer mit einer amorphen Struktur. Es besitzt keinen Schmelzpunkt, sondern nur eine Glasübergangstemperatur, die je nach Polymerisationsgrad zwischen 110 und 180 °C liegt. Erfindungsgemäß können Polyvinylpyrrolidone aller Polymerisationsgrade eingesetzt werden.

Bevorzugt sind Polyvinylpyrrolidone eines mittleren Molekulargewichts im Bereich von 10.000 bis 360.000 Dalton. Als geeignet haben sich beispielsweise mittlere Molekulargewichte von 15.000 Dalton und insbesondere 40.000 Dalton (Povidon K 30) erwiesen.

Zusätzlich zu den Hydroxypercarbonsäuren können weitere Percarbonsäuren enthalten sein, die in wässrige Lösung gebracht werden können. Dies sind beispielsweise gesättigte und ungesättigte aliphatische sowie aromatische Monocarbonsäuren mit bis zu 12 C-Atomen, etwa Peressigsäure, Perpropionsäure, Persorbinsäure und Perbenzoesäure. Hydroxypercarbonsäuren sind insbesondere solche mit bis zu 8 C-Atomen, wie Permilchsäure, Perzitronensäure, Peräpfelsäure und Perweinsäure sowie beliebige Mischungen derselben. Die Persäure kann über mehrere Hydroxy- und/oder Säurefunktionen verfügen. Insbesondere multifunktionelle Percarbonsäuren, wie Perzitronensäure, haben sich für erfindungsgemäße Zwecke als sehr effizient erwiesen.

Der Gehalt an mit PVP stabilisierter Hydroxypercarbonsäure (PVP plus Hydroxypercarbonsäure) in dem erfindungsgemäßen Mittel beträgt zweckmäßigerweise 0,1 bis 50 Gew.-%, vorzugsweise 1,0 bis 25 Gew.-%. Das Gewichtsverhältnis PVP zu Hydroxypercarbonsäure beträgt dabei 1:10 bis 10:1, vorzugsweise 1:5 bis 5:1. Naturgemäß ist die Wirksamkeit des Mittels umso größer, je höher die Percarbonsäurekonzentration ist, jedoch reichen für viele medizinische Einsatzzwecke bereits Konzentrationen um 1 Gew.-% aus. In der Regel wird zunächst ein Konzentrat hergestellt, das über eine Percarbonsäurekonzentration zwischen 10 und 20 Gew.-% verfügt, dessen Konzentration anschließend durch Verdünnung mit Wasser oder einem Lösungsmittel herabgesetzt wird.

Als vorteilhaft hat es sich erwiesen, in das Mittel mindestens zwei Percarbonsäuren einzubringen, da hier häufig synergistische Effekte beobachtet werden. So hat sich herausgestellt, dass unterschiedliche Percarbonsäuren sich für unterschiedliche Zwecke eignen. Beispielsweise werden eine Reihe von Toxinen, die über eine Aldehyd- oder Ketofunktion verfügen, mittels Perbenzoesäure über eine Baeyer-Villiger-Oxidation zerstört. Perbenzoesäure ist ferner gegen Aflatoxine einsetzbar und wirkt als Katalasehemmer. Peressigsäure greift die Porenproteine von Bakterien an, während Perzitronensäure besonders für den Abbau von Biofilmen geeignet ist. Persorbinsäure kann in Lipidschichten eindringen, auf diese Weise Zellmembranen durchdringen und Bakterien desaktivieren.

Das erfindungsgemäße Mittel kann zusätzlich zu der mit PVP stabilisierten Hydroxypercarbonsäure in wässriger Lösung ein mit Percarbonsäuren nicht reaktives Tensid enthalten, um die Netzfähigkeit zu erhöhen. In dieser Hinsicht bevorzugt sind Polysorbate in einer Menge von 0,5 bis 5 Gew.-%, beispielsweise Tween 20 oder Natriumdodecylsulfat.

Das erfindungsgemäße Mittel kann übliche Zusatzstoffe enthalten, insbesondere Lösemittel, Sequestrierungsmittel, pH-Regulatoren, weitere Persäurestabilisatoren, Komplexbildner, Entschäumungsmittel, Verdickungsmittel und Pflegemittel. Insbesondere kann das Mittel ein Feuchthaltemittel enthalten, beispielsweise Harnstoff oder langkettige Alkohole. Es versteht sich, dass alle diese Zusatzstoffe gegenüber Percarbonsäuren stabil sein müssen und auch den Zerfall der Percarbonsäuren nicht fördern.

Als Lösemittel dient in erster Linie Wasser. Das Mittel kann zudem organische Lösungsmittel enthalten, insbesondere Ethanol und Isopropanol. Alkohol weisen den Vorteil auf, dass sie einen zusätzlichen bakteriziden und fungiziden Effekt aufweisen, wodurch die keimtötende Wirkung der erfindungsgemäßen Zusammensetzung verstärkt wird.

Für die Verwendung als Desinfektionsmittel von Körperoberflächen, also Haut und Schleimhaut, ist das Mittel vorzugsweise als Spray, Gel, Creme oder Lotion formuliert. Auch hier gilt, dass die Formulierungshilfsstoffe oxidationsstabil sein müssen.

Der pH-Wert der wässrigen Lösung sollte im sauren Bereich liegen. Percarbonsäuren neigen in alkalischem Milieu zur Zersetzung. Typischerweise liegt der pH-Wert der für Anwendung der verdünnten Lösung zwischen 3 und 7, insbesondere zwischen 4 und 6. Gegebenenfalls kann der pH-Wert durch Einsatz von pH-Regulatoren eingestellt werden.

Das erfindungsgemäße Mittel hat sich als hochwirksam gegen Bakterien-, Virus- und Pilzinfektionen erwiesen. Darüber hinaus ist es auch geeignet, Stoffwechselprodukte von Mikroorganismen zu zerstören. Dies gilt sowohl für toxische Stoffwechselprodukte (Biotoxine) von Bakterien als auch für Adhäsine, also bakterielle Produkte, die die Anhaftung und das Wachstum von Bakterien an den Körper oder an Wunden fördern. Entsprechend das erfindungsgemäße Mittel als Desinfektions-und Reinigungsmittel für die Haut und Schleimhaut geeignet.

Bei der Bekämpfung von Mikroorganismen bewirkt die Freisetzung von Sauerstoffradikalen eine schnelle Oxidation der Außenhülle/Zellmembran. Auch Enzyme und andere Proteine sowie Nukleinsäuren werden durch die Percarbonsäuren angegriffen und geschädigt durch die daraus folgende Blockierung der Stoffwechselwege (Energiestoffwechsel, Proteinbiosynthese) tritt rasch der Zelltod ein daneben werden durch die erfindungsgemäße Zusammensetzung auch Biotoxine - Aflatoxine, Endotoxine, Mykotoxine und Allergene - zerstört.

Die Hydroxypercarbonsäuren des erfindungsgemäßen Mittels sind in der Lage, die Tertiärstruktur von Proteinen zu verändern, sodass diese ihre Eigenschaften verlieren. So werden Disulfidbrücken durch Oxidation aufgebrochen. Thiolgruppen werden zu Sulfonsäuren oxidiert.

Die erfindungsgemäßen Zusammensetzungen sind auch in der Lage, die Stoffwechselzentren von Mikroorganismen durch Oxidation der Enzyme, welche meist Schwermetalle wie beispielsweise Kupfer enthalten, zu deaktivieren. Viren können dadurch deaktiviert werden, dass die Kapsidproteine und Envelopeproteine oxidiert werden. Dies führt zu einer Desintegration des Nukleokapsids oder zu einer Veränderung der Kapsidoberfläche, die Aktivität des Virus zerstört.

Bei der Oxidation und der damit verbundenen Zerstörung von schädlichen Mikroorganismen wandelt sich die Percarbonsäure wieder zur Carbonsäure selbst um, sodass letztlich nur vergleichsweise harmlose Produkte entstehen. Es versteht sich, dass das erfindungsgemäße Mittel nicht nur zur Behandlung von Hauterkrankungen des Menschen herangezogen werden kann, sondern seine Wirksamkeit auch an tierischen Körper entfaltet.

Das erfindungsgemäße Mittel kann insbesondere auch zur Reinigung und Desinfektion von Wunden dienen, wie auch zur Behandlung von Fuß-und Nagelpilz, gegen Warzen und als Spülmittel bei der Mund-und Zahnhygiene. Bezüglich weiterer Einsatzgebiete wird ausdrücklich auf die WO 2006/125 657 A2 verwiesen.

Das erfindungsgemäße Mittel kann analog zu den in der WO 2006/125 657 A2 beschriebenen Prinzipien hergestellt werden. Als Ausgangsmaterialien kommen neben den jeweiligen Carbonsäuren auch deren Anhydride und Salze in Frage. Nachstehende Beispiele erläutern das Verfahren.

### Beispiel 1

In 90 g einer Wasserstoffperoxidlösung (20%) in Wasser werden zunächst 5 g Zitronensäure in Gegenwart einer katalytischen Menge Phosphorsäure bei Raumtemperatur zu Perzitronensäure umgesetzt. Nach der Gleichgewichtseinstellung wird die Mischung mit 5 g PVP K30 versetzt. Diese Mischung wird dann mit der zehnfachen Menge Wasser auf Anwendungsstärke verdünnt.

Die Gleichgewichtseinstellung der Mischungen kann einige Tage dauern. Ein Überschuss an Wasserstoffperoxid verschiebt das Gleichgewicht hin zur Percarbonsäure. Zur Beschleunigung kann der Mischung eine geringe Menge einer starken Säure, beispielsweise Methansulfonsäure, Phosphorsäure oder Schwefelsäure als Katalysator beigemischt werden.

Die so erhaltene Mischung ist zur Behandlung und Desinfektion von Hautwunden geeignet. Zur Herstellung eines Gels kann ein übliches Verdickungsmittel zugesetzt werden, etwa ein Acrylat.

### Beispiel 2

Ein gegen Warzen aktives Mittel wird wie folgt hergestellt:
90 g einer Wasserstoffperoxidlösung in Wasser (35%) werden vorgelegt und bei Raumtemperatur mit 1 g Benzoesäure, 3 g Zitronensäure und 1 g Natriumdiacetat versetzt. Die Mischung wird bis zur Einstellung des Reaktionsgleichgewichts gerührt und anschließend mit 5 g PVP K30 versetzt. Nach weiterem Rühren werden 900 ml deionisiertes Wasser, danach weitere 20 g PVP K30 zugegeben. Weitere Verdünnung mit 1000 ml deionisiertem Wasser ergibt das fertige Mittel.

## Patentansprüche

1. Mittel zur Anwendung auf Körperoberflächen zur Desinfektion und zum Zerstören von bakteriellen Stoffwechselprodukten, enthaltend wenigstens eine Hydroxypercarbonsäure zusammen mit einem Stabilisierungsmittel in wässriger Lösung, **dadurch gekennzeichnet, dass** das Stabilisierungsmittel Polyvinylpyrrolidon (PVP) ist.

2. Mittel nach Anspruch 1, **dadurch gekennzeichnet, dass** das PVP ein mittleres Molekulargewicht von 10.000 bis 360.000 Dalton hat.

3. Mittel nach Anspruch 2, **dadurch gekennzeichnet, dass** das PVP ein mittleres Molekulargewicht von 40.000 Dalton hat.

4. Mittel nach einem der Ansprüche 1 bis 3, **dadurch gekennzeichnet, dass** die Hydroxypercarbonsäure Permilchsäure, Perzitronensäure, Peräpfelsäure oder Perweinsäure ist.

5. Mittel nach einem der vorstehenden Ansprüche, **dadurch gekennzeichnet, dass** das Gewichtsverhältnis Hydroxypercarbonsäure zu PVP im Bereich von 1:10 bis 10: 1 liegt.

6. Mittel nach einem der vorstehenden Ansprüche, **dadurch gekennzeichnet, dass** die wässrige Lösung 0,1 bis 50 Gew.-%, vorzugsweise 1 bis 25 Gew.-% mit PVP stabilisierte Hydroxypercarbonsäure enthält.

7. Mittel nach einem der vorstehenden Ansprüche, **dadurch gekennzeichnet, dass** es weiterhin ein oxidationsstabiles Tensid enthält.

8. Mittel nach Anspruch 7, **dadurch gekennzeichnet, dass** es 0, 5 bis 5 Gew.-% Polysorbat enthält.

9. Mittel nach einem der vorstehenden Ansprüche, **dadurch gekennzeichnet, dass** es weiterhin ein Feuchthaltemittel enthält.

10. Zusammensetzung in Form eines Sprays, eines Gels, einer Creme oder einer Lotion, enthaltend das Mittel nach einem der Ansprüche 1 bis 9.

## Claims

1. Agent for use on body surfaces for disinfection and for destroying bacterial metabolic products, containing at least one percarboxylic acid together with a stabilising agent in aqueous solution, **characterised in that** the stabilising agent is polyvinylpyrrolidone (PVP).

2. Agent according to claim 1, **characterised in that** the PVP has an average molecular weight of 10,000 to 360,000 daltons.

3. Agent according to claim 2, **characterised in that** the PVP has an average molecular weight of 40,000 daltons.

4. Agent according to one of claims 1 to 3, **characterised in that** the percarboxylic acid is perlactic acid, percitric acid, permalic acid or pertartaric acid.

5. Agent according to any one of the preceding claims, **characterised in that** the weight ratio of percarboxylic acid to PVP is in the range from 1:10 to 10:1.

6. Agent according to any one of the preceding claims, **characterised in that** the aqueous solution contains 0.1 to 50% by weight, preferably 1 to 25% by weight, percarboxylic acid stabilised with PVP.

7. Agent according to any one of the preceding claims, **characterised in that** it also contains an oxidation-stable surfactant.

8. Agent according to claim 7, **characterised in that** it contains 0.5 to 5% by weight polysorbate.

9. Agent according to any one of the preceding claims, **characterised in that** it also contains a moistening agent.

10. Composition in the form of a spray, a gel, a cream or a lotion, containing the agent according to any one of claims 1 to 9.

## Revendications

1. Agent destiné à être utilisé sur des surfaces du corps pour désinfecter et détruire des métabolites bactériens, contenant au moins un peracide hydroxycarboxylique conjointement avec un agent de stabilisation dans une solution aqueuse, **caractérisé en ce que** l'agent de stabilisation est de la polyvinylpyrrolidone (PVP).

2. Agent selon la revendication 1, **caractérisé en ce que** la PVP a un poids moléculaire moyen de 10.000 à 360.000 Daltons.

3. Agent selon la revendication 2, **caractérisé en ce que** la PVP a un poids moléculaire moyen de 40.000 Daltons.

4. Agent selon l'une quelconque des revendications 1 à 3, **caractérisé en ce que** le peracide hydroxycarboxylique est du peracide lactique, du peracide citrique, du peracide malique ou du peracide tartrique.

5. Agent selon l'une quelconque des revendications précédentes, **caractérisé en ce que** le rapport de poids entre le peracide hydroxycarboxylique et la PVP se situe dans la plage de 1:10 à 10:1.

6. Agent selon l'une quelconque des revendications précédentes, **caractérisé en ce que** la solution aqueuse contient 0,1 à 50 % en poids, de préférence 1 à 25 % en poids de peracide hydroxycarboxylique stabilisé avec de la PVP.

7. Agent selon l'une quelconque des revendications précédentes, **caractérisé en ce qu'**il contient par ailleurs un tensioactif présentant une stabilité à l'oxydation.

8. Agent selon la revendication 7, **caractérisé en ce qu'**il contient 0,5 à 5 % en poids de polysorbate.

9. Agent selon l'une quelconque des revendications précédentes, **caractérisé en ce qu'**il contient par ailleurs un agent humectant.

10. Composition sous la forme d'un spray, d'un gel, d'une crème ou d'une lotion, contenant l'agent selon l'une quelconque des revendications 1 à 9.
